# EUROPÄISCHE PATENTSCHRIFT

(11) **EP 0 671 380 B1**
(45) Veröffentlichungstag und Bekanntmachung des Hinweises auf die Patenterteilung: **29.10.1997**
(21) Anmeldenummer: 95102943.8
(22) Anmeldetag: 02.03.1995
(51) Int. Cl.: C07C 67/32, C07C 253/30, C07D 315/00, C07C 69/24, C07C 69/75

(54) **Verfahren zur katalytischen Abspaltung von Carbonsäureester- und Acylgruppen aus organischen Verbindungen**
Process for the catalytic splitting off of carboxylic ester and acyl groups from organic compounds
Procédé d'élimination catalytiques de groupement esters carboxyliques et acyliques de composés organiques

(30) Priorität: 07.03.1994 DE 4407494
(43) Veröffentlichungstag der Anmeldung: 13.09.1995
(73) Patentinhaber: BASF AKTIENGESELLSCHAFT, 67056 Ludwigshafen (DE)
(72) Erfinder: Fischer, Rolf, Dr., D-69121 Heidelberg (DE)

(56) Entgegenhaltungen:
- EP-A- 0 055 797
- EP-A- 0 602 515
- DE-A- 2 638 453
- US-A- 3 299 100
- US-A- 4 422 979

## Beschreibung

Die vorliegende Erfindung betrifft ein Verfahren zur Herstellung von Verbindungen der allgemeinen Formel I 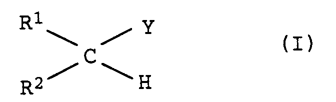 in der die Reste folgende Bedeutung haben:
- Y: 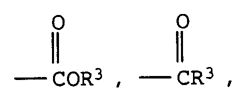 -CN, -SO₂R³, wobei R³ für Wasserstoff oder einen C₁-C₁₀-Kohlenwasserstoffrest steht,
gewünschtenfalls inerte Substituenten tragendes Phenyl,
- R¹,R²: unabhängig voneinander Wasserstoff, gewünschtenfalls inerte Substituenten tragender C₁-C₂₀-Kohlenwasserstoffrest,
Heteroaryl, weiterhin C₁-C₆-Acyl, wenn Y für 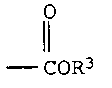 steht,
oder gemeinsam eine C₁-C₁₂-Alkylenbrücke, die als Brückenglied Sauerstoff, Schwefel oder NR⁴ enthalten kann, wobei R⁴ für Wasserstoff oder einen C₁-C₄-Alkylrest steht.

Geminale Dicarbonsäurediester können in dreistufiger Reaktion durch alkalische Verseifung zur entsprechenden geminalen Dicarbonsäure, anschließend durch thermische Abspaltung einer Carboxylgruppe und Veresterung der verbleibenden Carboxylgruppe in Monocarbonsäureester umgewandelt werden (JP-A-62/201 843; Chem. Abstr. Vol. 109, 14925).

Weiterhin ist bekannt, aus geminalen Dicarbonsäurediestern durch Erhitzen mit Gemischen aus Mineralsäuren und Carbonsäuren entsprechende Monocarbonsäuren zu erhalten (Arch. Pharm. 319, 29 (1986)), die anschließend zu den gewünschten Monocarbonsäureestern verestert werden müssen.

In Tetrahedron Lett. 27, 2283 (1986) wird gelehrt, geminale Dicarbonsäurediester einstufig durch Erhitzen in polaren Lösungsmitteln wie Dimethylsulfoxid oder Dimethylformamid in Gegenwart von Alkalichloriden oder -cyaniden in Monocarbonsäureester umzuwandeln.

Schließlich lassen sich geminale Dicarbonsäurediester nach der Lehre der EP-A 546 395 an oxidischen Katalysatoren, bevorzugt in der Gasphase bei Temperaturen von 250-350 °C, einstufig zu Monocarbonsäureestern umsetzen.

Die beiden ersten Methoden zur Herstellung von Carbonsäureestern aus geminalen Dicarbonsäureestern besitzen den Nachteil, daß mehrere Reaktionsstufen erforderlich sind und daß im ersten Fall Salzanfall auftritt. Für die dritte, einstufige Synthese sind sehr lange Reaktionszeiten erforderlich. Die vierte Methode, die bei hohen Temperaturen durchgeführt wird, setzt die Verdampfbarkeit und die thermische Beständigkeit der Ausgangsverbindung voraus, so daß sie nicht für alle gewünschten Reaktionsprodukte anwendbar ist.

Acetylgruppen substituierter Butyrolactone und Butanlactame lassen sich nach dem in der EP-A 0 602 515 beschriebenen Verfahren durch Erhitzen mit einem Überschuß an Dimethylcarbonat oder Ethylencarbonat in Gegenwart von stickstoffhaltigen Basen durch Methyl- oder Hydroxymethylgruppen substituieren.

Acylgruppen in Verbindungen, die sich von Acetessigsäureestern ableiten, lassen sich durch Erhitzen mit alkoholischen Alkanolat-Lösungen abspalten (F.D. Gunstone, Q. Rev. Chem. Soc. 7, 175 (1953) und W.J. Gensler, Chem. Rev. 57, 191 (1957)).

In ähnlicher Weise lassen sich nach der US-A 4 422 979 Estergruppen aus durch Alkyl- und Phenylreste substituierten Cyanessigsäureestern eliminieren.

Da die Alkanolate bei der Aufarbeitung der Reaktionsausträge hydrolysiert werden müssen, sind diese beiden Methoden mit Salzanfall verbunden.

Es bestand daher die Aufgabe, ein Verfahren bereitzustellen, das die genannten Nachteile bei der Abspaltung von Acyl- und Carbonsäureestergruppen aus organischen Verbindungen vermeidet.

Demgemäß wurde das eingangs definierte Verfahren zur Herstellung von Verbindungen der Formel I gefunden, das dadurch gekennzeichnet ist, daß man eine Verbindung der allgemeinen Formel II 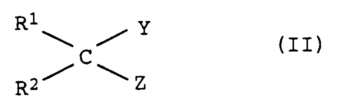 in der Z 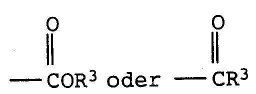 bedeutet, in Gegenwart katalytischer Mengen eines Kohlensäureesters und einer stickstoffhaltigen Base umsetzt.

Vorteilhaft gegenüber der Verwendung von Alkoholaten in Alkoholen als Lösungsmitteln ist, daß die erfindungsgemäß verwendeten Einsatzstoffe - Kohlensäurediester und stickstoffhaltige Basen - nicht wie Alkoholate leicht hydrolysiert werden und daß daher nicht unter extremem Feuchtigkeitsausschluß gearbeitet werden muß. Eine Aufarbeitung mit Wasser führt nicht wie im Falle der Alkoholate zu Mineralsalze enthaltenden Abwässern.

Das erfindungsgemäße Verfahren läßt sich für den Fall der Abspaltung einer Estergruppe aus einem geminalen Dicarbonsäurediester wie Dimethylmalonsäuredimethylester mit Dimethylcarbonat, Ethyldimethylamin und Methanol durch die Reaktionsgleichung 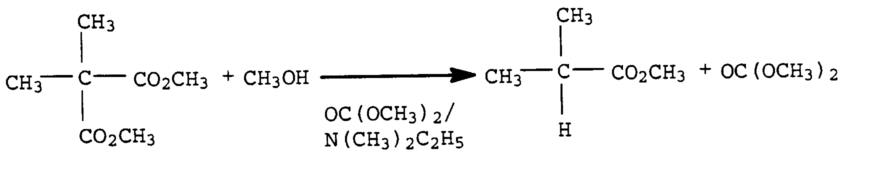 darstellen.

Die Reste der Verfahrensprodukte I und der Ausgangsverbindungen II sind oben definiert. Im einzelnen handelt es sich um:
- Y: Der Rest Y steht für Carbonsäuregruppen oder Carbonsäureestergruppen wie Methoxycarbonyl, Ethoxycarbonyl, weiterhin für Formyl, Acyl wie Acetyl, Propanoyl, außerdem für Cyano, für einen Sulfonsäureester oder Alkylsulfonylreste wie Methylsulfonyl, für einen Phenylrest, der ein bis drei inerte Substituenten tragen kann, beispielsweise Halogen wie Fluor, Chlor, Brom und Jod, C₁-C₆-Alkoxyreste wie Methoxy und Ethoxy, Cyano, Nitro.
- Z: Bevorzugt sind solche Ausgangsverbindungen, in denen Z für eine Carbonsäureestergruppe und Y für eine Carbonsäureester-, Cyano- oder Acylgruppe steht.
- R¹, R²: Außer für Wasserstoff stehen diese Reste für C₁-C₂₀-Kohlenwasserstoffreste wie geradkettige oder verzweigte Alkylgruppen mit bevorzugt 1 bis 6 Kohlenstoffatomen, z.B. Methyl, Ethyl, n-Propyl, iso-Propyl, n-Butyl, sec-Butyl. Diese Gruppen können inerte Substituenten wie Halogenatome tragen. Aber auch Carbonsäureestergruppen kommen als solche inerte Substituenten in Betracht wie im Rest Methoxycarbonylmethyl.
Weiterhin kommen Cycloalkylgruppen mit bevorzugt 3 bis 8 Kohlenstoffatomen wie Cyclopropyl, Cyclobutyl, Cyclopentyl, Cyclohexyl in Betracht.
Als Kohlenwasserstoffreste sind weiterhin Biphenyl und Arylreste wie Phenyl zu nennen.
Außerdem kommen Aralkylreste wie Benzyl in Betracht, die ebenfalls am aromatischen Ring, wie oben für den Rest Y beschrieben, substituiert sein können. Als Heteroarylreste sind Thienyl- und Pyridylreste zu nennen.

Weiterhin können R¹ und R² eine gemeinsame C₁-C₁₂ -Alkylenbrücke bilden, wie in Sulfolan, welche gegebenenfalls Heteroatome enthalten kann.

Wenn Y für eine Carbonsäure- oder Carbonsäureestergruppe steht, kann R¹ bzw. R² für C₂- bis C₆-Acyl stehen, z.B. Acetyl wie in 2-Acetylmalonsäuredimethylester.

Die Reste R³ in den Substituenten Y und Z können gleich oder verschieden sein.

Bevorzugte Ausgangsverbindungen der Formel II sind z. B. in 2-Stellung substituierte Malonsäurediester wie 2-Methylmalonsäuredimethylester, 2-Ethylmalonsäurediethylester, 2-Acetylmalonsäuredimethylester, 2-Phenylmalonsäurediethylester, 2,2-Dimethylmalonsäuredimethylester, 2,2-Diacetylmalonsäuredipropylester, 2-Benzylmalonsäuredimethylester, weiterhin 1,1-Cyclopropandicarbonsäuredimethylester, 1,1-Cyclohexandicarbonsäurediethylester, Tetrahydropyran-4,4-dicarbonsäuredimethylester, Tetrahydrothiopyran-4,4-dicarbonsäuredimethylester, 1,1-Cyclopentandicarbonsäuredimethylester, 2,2-Dimethylcyanessigsäuremethylester, 2-Methyl-2-n-butylcyanessigsäuremethylester, 2-Formylbernsteinsäuredimethylester, 2,2-Dimethylacetessigsäuremethylester, 3-Methyl-3-butylacetylaceton, 2-Methyl-2-(o-methoxy-phenyl)-cyanessigsäureethylester, 2-Methyl-2-(o-fluorobiphenylyl)-cyanessigsäureethylester, 2-Thienylcyanessigsäureethylester, 2-Methyl-2-(m-ethoxyethylphenyl)-cyanvaleriansäureethylester, 2-Methyl-2-(m-chlor-p-methoxyphenyl)-malonsäuredimethylester, 2-Methyl-2-carbomethoxysulfolan, 2-Carbomethoxysulfolan.

Die für die erfindungsgemäße Umsetzung benötigten Verbindungen II sind handelsüblich oder nach bekannten Methoden herstellbar, z.B. durch Mono- oder Bisalkylierung oder Acylierung von Malonsäurediestern, Cyanessigsäureestern, Acetessigsäureestern und Phenylacetonitrilen.

Das erfindungsgemäße Verfahren läßt sich wie folgt durchführen:

Die Verbindungen der allgemeinen Formel II können in Gegenwart von stickstoffhaltigen Basen bei Temperaturen von 100 bis 250 °C, bevorzugt 130 bis 230 °C, besonders bevorzugt 150 bis 210 °C und Drücken von 0,01 bis 100 bar, bevorzugt 5 bis 50 bar, besonders bevorzugt bei dem sich im jeweiligen Reaktionsgemisch einstellenden Druck umgesetzt werden.

Die Umsetzung kann in der Flüssigphase diskontinuierlich oder kontinuierlich durchgeführt werden, beispielsweise in einem Autoklaven.

Es kann bei der Herstellung hydrolyseempfindlicher Produkte vorteilhaft sein, die Umsetzung in Gegenwart von unter den Reaktionsbedingungen inerten Gasen wie Stickstoff oder Argon durchzuführen.

Die Herstellung der Verbindungen I in der Flüssigphase kann beispielsweise so durchgeführt werden, daß man eine Verbindung II in Gegenwart von Kohlensäurediestern und einer stickstoffhaltigen Base auf die gewünschte Reaktionstemperatur erhitzt. Nach beendeter Reaktion kann das Reaktionsgemisch abgekühlt und zur Gewinnung der gewünschten Verbindungen I fraktionierend destilliert werden.

Die erfindungsgemäße Umsetzung kann in Abwesenheit von Lösungsmitteln durchgeführt werden. Es kann jedoch vorteilhaft sein, in Anwesenheit von Lösungsmitteln zu arbeiten. Als Lösungsmittel können beispielsweise acyclische oder cyclische Ether wie Diethylether, Tetrahydrofuran und Dioxan, aromatische Kohlenwasserstoffe wie Benzol, Toluol und Xylol, chlorierte Kohlenwasserstoffe wie Chloroform und Methylenchlorid verwendet werden.

Die Menge an Lösungsmittel beträgt, bezogen auf die eingesetzten Verbindungen II 0 bis 90 Gew.-%, bevorzugt 0 bis 30 Gew.-%.

Als Kohlensäurediester kommen Dialkylester wie z. B. Dimethl-, Diethyl-, Diisopropyl-, Di-n-butyl- und Diallylcarbonate, Diarylester wie Diphenyl-, Ditolylcarbonat, cyclische Ester wie Ethylen- und Propylencarbonat, Dibenzylcarbonat, Cycloalkylester wie Dicyclohexylcarbonat und gemischte Ester mit aliphatischen und aromatischen Resten wie Phenylallyl-, Ethylphenylcarbonat in Frage, wie sie z. B. in Kirk-Othmer, Encyclopedia of Chemical Technology, Vierte Auflage, Band 5, Seiten 87-88 (1993) beschrieben sind.

Als stickstoffhaltige Basen eignen sich z.B. Ammoniak oder Amine mit 1 bis 30 Kohlenstoffatomen wie primäre, sekundäre und tertiäre Amine mit aliphatischen, cycloaliphatischen, heteroaromatischen und/oder araliphatischen Substituenten. Dabei können zwei Substituenten auch zu einem Ring geschlossen sein. Weiterhin kommen funktionelle Gruppen wie Hydroxygruppen tragende Amine, aromatische Amine und Polyamine in Betracht.

### Beispiele hierfür sind:

- Ammoniak,
- Methylamin, Ethylamin, Hexylamin und Cyclohexylamin,
- Dimethylamin, Diethylamin, Dibutylamin und Dicyclohexylamin,
- Trimethylamin, Dimethylethylamin, Triethylamin, Tri-n-propylamin, Triisopropylamin, Tributylamin, Trioctylamin, Tricyclohexylamin, Trihexyldecylamin, Tricyclohexylamin, Diphenylmethylamin, Dimethylbenzylamin, Dibenzylmethylamin, Tribenzylamin,
- N,N-Tetramethylhexamethylendiamin, Hexamethylendiamin, Tetramethylendiamin,
- Ethanolamin, Diethanolamin, Triethanolamin,
- 4-Dimethylaminopyridin, Urotropin, Piperidin, N-Methylpiperidin, Pyrrolidin, N-Methylpyrrolidin, Hexamethylenimin, N-Ethylhexamethylenimin, N-Methylimidazol, 1,4-Diazabicyclo[4.3.0]octan (DABCO), Morpholin, Piperazin, Pyrrolidin, 2,6-Dimethylmorpholin.

Weiterhin eignen sich Amidine wie 1,5-Diazabicyclo[4.3.0]non-5-en (DBN), 1,8-Diazabicyclo-[5.4.0]undec-7-en (DBU) und Guanidin.

Bevorzugt sind tertiäre Amine, besonders bevorzugt C₃- bis C₂₄-Trialkylamine. Weiterhin bevorzugt sind Amidine wie DBN und DBU.

Das Molverhältnis der Verbindungen II zu den Kohlensäurediestern und den stickstoffhaltigen Basen beträgt in der Regel 1 : 0,01 : 0,01 bis 1 : 0,5 : 0,5, bevorzugt 1 : 0,05 : 0,05 bis 1 : 0,2 : 0,2. Im allgemeinen werden die Kohlensäurediester und die stickstoffhaltigen Basen dem Reaktionsgemisch etwa in äquimolarem Verhältnis zugesetzt.

Es hat sich als vorteilhaft erwiesen, das erfindungsgemäße Verfahren in Gegenwart eines Alkohols durchzuführen.

Als Alkohole können z. B. aliphatische, cycloaliphatische, araliphatische ein- und mehrwertige Alkohole und Phenole, wie Methanol, Ethanol, n-Propanol, i-Propanol, Butanole, Decanole, Dodecanole, Ethylenglykol, 1,4-Butandiol, 1,3-Propandiol, Glycerin, 1,8-Octandiol, Cyclohexanol, Cyclopentanol, 1,4-Cyclohexandiol, Benzylalkohol, Phenol verwendet werden, bevorzugt werden C₁-C₆-Alkanole. Um bei der Abspaltung von Estergruppen aus geminalen Dicarbonsäurediestern einheitliche Monoester zu erhalten, kann es sinnvoll sein, diejenigen Alkohole zu verwenden, die in den Estergruppen enthalten sind.

Das Molverhältnis der Verbindungen II zu den Alkoholen beträgt in der Regel 1 : 0,1 bis 1 : 5, insbesondere 1 : 0,5 bis 1 : 1,5.

Die Reaktionszeiten liegen in der Regel bei 0,1 bis 10 Stunden.

Die Verfahrensprodukte dienen z.B. als Vorprodukte für Pharmazeutika und Pflanzenschutzmittel.

### Beispiele

### Allgemeine Herstellvorschrift für die Beispiele 1 - 7

Gemische aus den Ausgangsverbindungen II (0,05 mol), Ethyldimethylamin 1, Dimethylcarbonat 2 und Methanol 3 wurden in einem Autoklaven unter Rühren auf 200 °C aufgeheizt und drei Stunden bei dieser Temperatur gerührt. Die Molverhältnisse der Reaktionspartner sind in Tabelle 1 zusammengestellt. Nach dem Abkühlen und Entspannen des Autoklaven wurde der Reaktionsaustrag gewogen. Die Reaktionsprodukte wurden durch GC-MS-Kopplung und Vergleich der GC-Retentionszeiten mit Vergleichssubstanzen identifiziert. Die Ausbeuten an Verbindungen I in Tabelle 1 wurden durch quantitative GC-Analyse ermittelt.

Bei Abspaltung von Carbonsäuremethylester-Gruppen entstand als Koppelprodukt Dimethylcarbonat, im Falle von Acetylgruppen Essigsäuremethylester. Diese Verbindungen wurden in Ausbeuten zwischen 44 und 74 % isoliert.

Folgende Produkte wurden hergestellt:

### Beispiel 1

2-Methylpropionsäuremethylester aus Dimethylmalonsäuredimethylester

### Beispiel 2

Cyclohexancarbonsäuremethylester aus Cyclohexan-1,1-dicarbonsäuredimethylester

### Beispiel 3

Propionsäuremethylester aus Methylmalonsäuredimethylester

### Beispiel 4

Tetrahydropyran-4-carbonsäuremethylester aus Tetrahydropyran-4,4-dicarbonsäuredimethylester

### Beispiel 5

2-Methylhexannitril aus 2-Methyl-2-butyl-cyanessigsäuremethylester

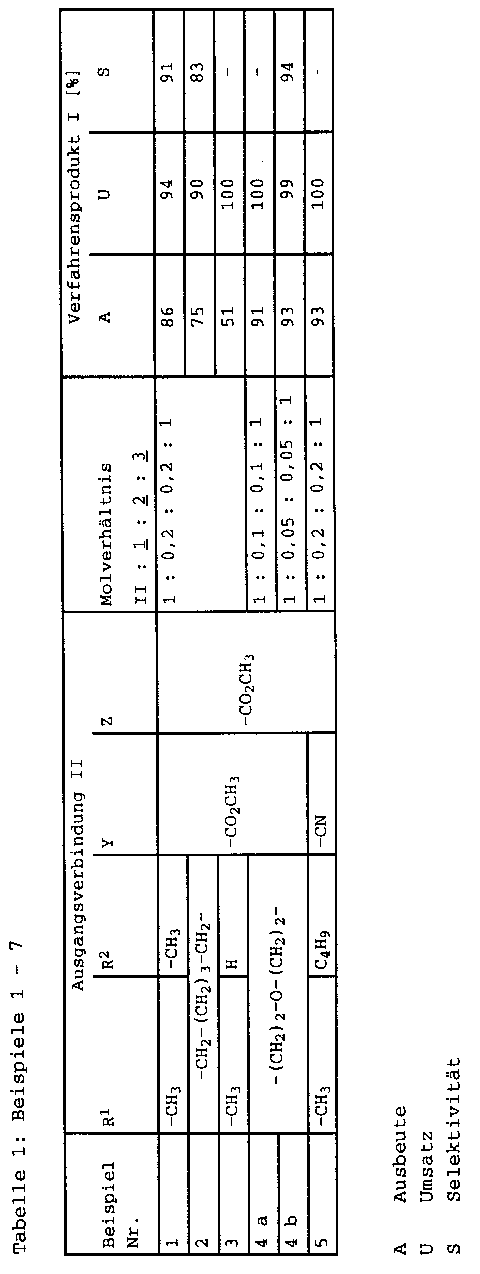

### Beispiel 8

Beispiel 1 wurde mit dem Unterschied wiederholt, daß anstelle von Ethyldimethylamin die gleiche Molmenge Triethylamin eingesetzt wurde. Die quantitative gaschromatographische Analyse ergab eine i-Buttersäuremethylester-Ausbeute von 88 %, der Umsatz betrug 96 %.

### Beispiel 9

Beispiel 1 wurde mit dem Unterschied wiederholt, daß anstelle von Ethyldimethylamin die gleiche Molmenge 1,8-Diazabicyclo-[5.4.0]undec-7-en (DBU) eingesetzt wurde. Die quantitative gaschromatographische Analyse ergab eine i-Buttersäuremethylester-Ausbeute von 94 %, der Umsatz war quantitativ.

### Beispiel 10

Beispiel 1 wurde mit dem Unterschied wiederholt, daß anstelle von Dimethylcarbonat die gleiche Molmenge Diethylcarbonat und anstelle von Methanol die gleiche Molmenge Ethanol eingesetzt wurde. Die quantitative gaschromatographische Analyse ergab eine i-Buttersäuremethylester-Ausbeute von 44 % und eine i-Buttersäureethylester-Ausbeute von 33 %. Der Umsatz betrug 85 %.

### Beispiel 11

Beispiel 1 wurde mit dem Unterschied wiederholt, daß anstelle von Dimethylcarbonat die gleiche Molmenge Diphenylcarbonat eingesetzt wurde. Die quantitative gaschromatographische Analyse ergab eine i-Buttersäuremethylester-Ausbeute von 90 %, der Umsatz betrug 95 %.

### Beispiel 12 (ohne Alkohol)

Ein Gemisch aus 6,8 g 2-(n-Butyl)-2-methyl-cyanvaleriansäuremethylester, 0,18 g Dimethylcarbonat, 0,15 g Ethyldimethylamin und 1,6 g Tetrahydrofuran wurde 3 Stunden lang auf 200 °C erhitzt. Die quantitative gaschromatographische Analyse ergab eine 2-Methylcapronitril-Ausbeute von 22 %, der Umsatz betrug 53 %.

## Patentansprüche

1. Verfahren zur Herstellung von Verbindungen der allgemeinen Formel I 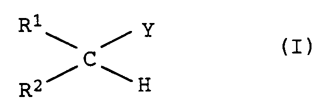 in der die Reste folgende Bedeutung haben:
Y 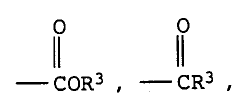 -CN, -SO₂R³, wobei R³ für Wasserstoff oder einen C₁-C₁₀-Kohlenwasserstoffrest steht,
gewünschtenfalls inerte Substituenten tragendes Phenyl,
R¹, R² unabhängig voneinander Wasserstoff, gewünschtenfalls inerte Substituenten tragender C₁-C₂₀-Kohlenwasserstoffrest, Heteroaryl, weiterhin C₁-C₆-Acyl, wenn Y für 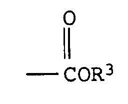 steht,
oder gemeinsam eine C₁-C₁₂-Alkylenbrücke, die als Brückenglied Sauerstoff, Schwefel oder NR⁴ enthalten kann, wobei R⁴ für Wasserstoff oder einen C₁-C₄-Alkylrest steht,
dadurch gekennzeichnet, daß man eine Verbindung der allgemeinen Formel II 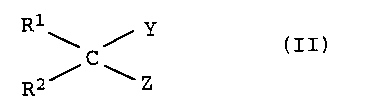 in der Z 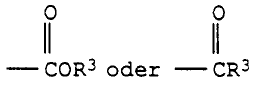 bedeutet, in Gegenwart katalytischer Mengen eines Kohlensäureesters und einer stickstoffhaltigen Base umsetzt.

2. Verfahren nach Anspruch 1, dadurch gekennzeichnet, daß man als Kohlensäureester Dimethylcarbonat verwendet.

3. Verfahren nach Anspruch 1 oder 2, dadurch gekennzeichnet, daß man als stickstoffhaltige Base C₃-C₂₄-Trialkylamine, 1,5-Diazabicyclo-[4.3.0]non-5-en (DBN) oder 1,8-Diazabicyclo-[5.4.0]undec-7-en (DBU) einsetzt.

4. Verfahren nach den Ansprüchen 1 bis 3, dadurch gekennzeichnet, daß das Molverhältnis der Verbindung II zum Kohlensäureester und zur stickstoffhaltigen Base 1 : 0,05 : 0,05 bis 1 : 0,2 : 0,2 beträgt.

5. Verfahren nach den Ansprüchen 1 bis 4, dadurch gekennzeichnet, daß man die Umsetzung bei 100 bis 250°C vornimmt.

6. Verfahren nach den Ansprüchen 1 bis 5, dadurch gekennzeichnet, daß man die Umsetzung in Gegenwart eines C₁-C₆-Alkanols vornimmt.

## Claims

1. A process for preparing compounds of the general formula I 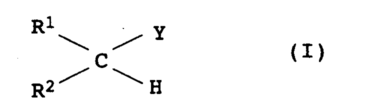 where :
Y is 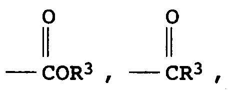 -CN, -SO₂R³, where R³ is hydrogen or a C₁-C₁₀ hydrocarbon radical,
phenyl, which carries inert substituents if desired,
R¹ and R² are each, independently of one another, hydrogen, a C₁-C₂₀ hydrocarbon radical which carries inert substituents if desired, or heteroaryl, furthermore C₁-C₆-acyl, when Y is 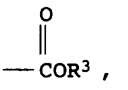 or together are a C₁-C₁₂-alkylene bridge which may contain as bridge member oxygen, sulfur or NR⁴ where R⁴ is hydrogen or C₁-C₄-alkyl,
which comprises reacting a compound of the general formula II 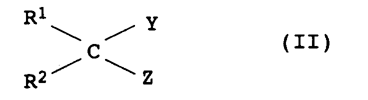 where Z is 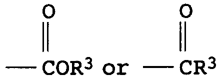 in the presence of catalytic amounts of a carbonic ester and of a nitrogenous base.

2. A process as claimed in claim 1, wherein dimethyl carbonate is used as carbonic ester.

3. A process as claimed in claim 1 or 2, wherein C₃-C₂₄-trialkylamines, 1,5-diazabicyclo[4.3.0]non-5-ene (DBN) or 1,8-diazabicyclo[5.4.0]undec-7-ene (DBU) is used as nitrogenous base.

4. A process as claimed in any of claims 1 to 3, wherein the molar ratio of the compound II to the carbonic ester and to the nitrogenous base is from 1 : 0.05 : 0.05 to 1 : 0.2 : 0.2.

5. A process as claimed in any of claims 1 to 4, wherein the reaction is carried out at from 100 to 250°C.

6. A process as claimed in any of claims 1 to 5, wherein the reaction is carried out in the presence of a C₁-C₆-alkanol.

## Revendications

1. Procédé de préparation de composés de formule générale 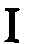 : 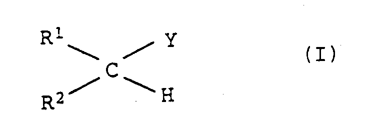 dans laquelle les restes ont la signification suivante :
Y 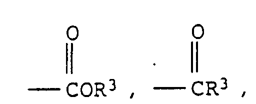 -CN, -SO₂R³, où R³ est mis pour un atome d'hydrogène ou un reste hydrocarboné en C₁-C₁₀, un reste phényle portant éventuellement des substituants inertes
R¹, R² indépendamment l'un de l'autre, un atome d'hydrogène, un reste hydrocarboné en C₁-C₂₀ portant éventuellement des substituants inertes, un reste hétéroaryle, un reste acyle en C₁-C₆, lorsque Y est mis pour 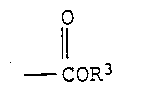 ou, ensemble, un pont alkylène en C₁-C₁₂, pouvant contenir de l'oxygène, du soufre ou NR⁴ en tant que maillons, où R⁴ est mis pour un atome d'hydrogène ou un reste alkyle en C₁-C₄,
caractérisé en ce que l'on fait réagir un composé de formule générale 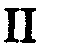 : 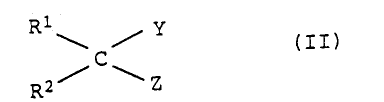 dans laquelle Z signifie 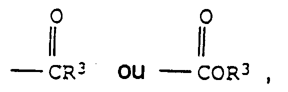 en présence de quantités catalytiques d'un ester d'acide carboxylique et d'une base contenant de l'azote.

2. Procédé selon la revendication 1, caractérisé en ce que l'on utilise du carbonate de diméthyle en tant qu'ester d'acide carboxylique.

3. Procédé selon la revendication 1 ou 2, caractérisé en ce que l'on utilise une trialkylamine en C₃-C₂₄, le 1,5-diazabicyclo-[4.3.0]-non-5-ène (DBN) ou le 1,8-diazabicyclo-[5.4.0]undèc-7-ène (DBU), en tant que base contenant de l'azote.

4. Procédé selon l'une quelconque des revendications 1 à 3, caractérisé en ce que le rapport en moles du composé 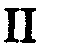 à l'ester d'acide carboxylique et à la base contenant de l'azote s'élève de 1:0,05:0,05 à 1:0,2:0,2.

5. Procédé selon l'une quelconque des revendications 1 à 4, caractérisé en ce que l'on effectue la réaction à 100-250°C.

6. Procédé selon l'une quelconque des revendications 1 à 5, caractérisé en ce que l'on effectue la réaction en présence d'un alcanol en C₁-C₆.
